# EUROPÄISCHE PATENTSCHRIFT

(11) **EP 1 844 125 B1**
(45) Veröffentlichungstag und Bekanntmachung des Hinweises auf die Patenterteilung: **27.05.2009**
(21) Anmeldenummer: 05810718.6
(22) Anmeldetag: 01.12.2005
(51) Int. Cl.: C10G 3/00, C10G 50/00, C07C 1/20, C07C 2/12

(54) **VERFAHREN ZUR HERSTELLUNG VON SYNTHETISCHEN KRAFTSTOFFEN AUS OXIGENATEN**
METHOD FOR THE PRODUCTION OF SYNTHETIC FUELS FROM OXYGENATES
PROCEDE DE PRODUCTION DE COMBUSTIBLES SYNTHETIQUES A BASE D'OXYGENATS

(30) Priorität: 22.01.2005 DE 102005003109
(43) Veröffentlichungstag der Anmeldung: 17.10.2007
(73) Patentinhaber: Lurgi AG, 60295 Frankfurt am Main (DE)
(72) Erfinder: ROTHÄMEL, Martin, 60437 Frankfurt-Niedererlenbach (DE); KÖMPEL, Harald, 63263 Neu-Isenburg (DE); AHLERS, Bernd, 61440 Oberursel (DE); HOFMOCKEL, Jürgen, 60314 Frankfurt am Main (DE); WAGNER, Matthias, 61130 Nidderau (DE)
(74) Vertreter: Meyer-Dulheuer, Karl-Hermann
(86) Internationale Anmeldenummer: PCT/EP2005/012789
(87) Internationale Veröffentlichungsnummer: WO 2006/076942

(56) Entgegenhaltungen:
- US-A- 4 579 999
- US-A- 4 689 205
- US-A- 5 672 800

## Beschreibung

Gegenstand der Erfindung ist ein mehrstufiges Verfahren zur Herstellung von synthetischen Kraftstoffen aus Oxigenaten wie Methanol und/oder Dimethylether, wobei die Dieselausbeute optimiert werden soll. Unter synthetischen Kraftstoffen werden Diesel, Kerojet und Benzin verstanden.

### HINTERGRUND DER ERFINDUNG

Es sind bereits mehrere Verfahren zur Herstellung von synthetischen Kraftstoffen entwickelt worden. Insbesondere sind auch bereits Verfahren bekannt geworden, die ausgehend von Methanol die Herstellung von synthetischen Kraftstoffen ermöglichen.

Prinzipiell sind diese Verfahren in zwei Verfahrensstufen aufzuteilen. Im der ersten Verfahrensstufe (Olefinerzeugung) werden Oxigenate mit Hilfe eines Zeolith-Katalysators vorzugsweise zu C₂ - C₈ Olefinen umgesetzt. In einer zweiten Verfahrensstufe (Oligomerisierung) werden die genannten Olefine bei erhöhtem Druck mit Hilfe eines Zeolith-Katalysators zu höheren Kohlenwasserstoffen umgesetzt. Zwischen den beiden Verfahrensstufen können Teilströme bestehend aus Wasser und/oder Kohlenwasserstoff in einem ersten Separationsschritt abgetrennt werden. Nach der 2. Verfahrensstufe werden die erhaltenen höheren Kohlenwasserstoffe in einer zweiten Separation in eine leichte Fraktion, eine Benzin-Fraktion und eine schwere Fraktion aufgetrennt. Die schwere Fraktion bildet nach Hydrierung das Endprodukt Diesel und bei geeigneter Separation auch Kerojet. Aus der leichten Fraktion können die Produkte LPG (Flüssiggas) und Heizgas gewonnen werden.

Die im Folgenden betrachteten bekannten Verfahren stellen Teilschritte des oben beschriebenen Gesamtverfahrens dar:

Die erste Verfahrensstufe (Olefinerzeugung) ist zum Beispiel bei der Herstellung von insbesondere Propylen aus Methanol in den europäischen Patentschriften EP 0 448 000 B1 und EP 0 882 692 B1, sowie in den deutschen Patentanmeldungen DE 100 27 159 A1 und DE 101 17 248 A1 beschrieben. Zum Einsatz kommt hier das sogenannte MTP-Verfahren (MTP = Methanol to Propylene). Dieses Verfahren verwendet einen mehrstufigen Festbettreaktor. Als Katalysator wird ein Zeolith des Pentasiltyps verwendet. Zur Optimierung der Produktausbeute wird ein Gasstrom zum Olefinreaktor rezirkuliert, der reich an ungesättigten Kohlenwasserstoffen ist, und der aus dem Reaktorprodukt dieser Verfahrensstufe abgetrennt wird. Zusätzlich wird das bei der Olefinerzeugung produzierte Wasser teilweise rezirkuliert. Das angestrebte Produkt dieser Verfahrensstufe ist beim MTP-Verfahren allerdings Propylen und nicht etwa Olefine.

Das Prinzip der ersten Verfahrensstufe (Olefinerzeugung) wird in anderen Patenten zur Erzeugung von Benzin eingesetzt. Zu nennen sind hier die US Patente 4,404,414 sowie 4,788,369 und 4,035,430. Die hier beschriebenen Verfahren arbeiten auch mit mehrstufigen Festbettreaktoren, die mit Zeolith Katalysator (ZSM 5 oder ähnlichen Katalysatoren) befüllt sind. Auch hier werden Gasströme zum Reaktor rezirkuliert, die je nach Patent aus gesättigten und ungesättigten Kohlenwasserstoffen bestehen. Durch die Rezirkulation soll das Temperaturprofil im Reaktor eingestellt werden und die Produktausbeute zu Benzin optimiert werden. Diese Patente verwenden allerdings kein Wasser als Rezirkulationsstrom, wie dies bei dem schon genannten MTP Verfahren der Fall ist.

Die zweite Verfahrensstufe (Oligomerisierung), in der aus Olefinen höhere Kohlenwasserstoffe oligomerisiert werden, die dann zu Dieselkraftstoff hydriert werden können, ist aus den südafrikanischen Patenten 9,101,969, 9,101,970 und 9,200,642 bekannt. Für die Oligomerisierung werden hier Katalysatoren auf der Basis von kristallinen Aluminiumsilikaten des Pentasiltyps eingesetzt, die insbesondere in der europäischen Patentschrift 369 364 beschrieben sind. Auch hier kommt ein mehrstufiger Festbettreaktor zur Anwendung. Eingesetzt wird dieses Verfahren vor allem zur Herstellung von Diesel aus Kohlenwasserstoffen, die vorzugsweise C₂ bis C₈ Olefine enthalten. Auch in diesem Verfahren wird zur Optimierung der Produktausbeute mit einem Rezirkulationsstrom gearbeitet.

Auch die Kombination eines Verfahrens zur Herstellung von Olefinen aus Methanol mit einem Verfahren zur Herstellung von Benzin und Diesel aus Olefinen ist bereits Gegenstand von mehreren Patenten.

Hierbei ist insbesondere auf die US-Patentschriften 4,579,999 und 4,689,205 sowie 4,898,717 hinzuweisen. Die vorstehend genannten Patente beschreiben ein zweistufiges Verfahren zur Herstellung von Diesel und Benzin aus Methanol. Zum Einsatz kommt hierbei das sogenannte MTO-Verfahren (MTO = Methanol to Olefins) in Kombination mit dem MOGD-Verfahren (MOGD = Mobil Olefins to Gasoline/Distillate), das die Oligomerisierung von Olefinen zum Gegenstand hat. Alle drei Patente setzen Katalysatoren vom Zeolith-Typ ein und beschreiben, unter welchen Betriebsbedingungen vorzugsweise der Kraftstoff Diesel herzustellen ist.

In den ersten beiden US-Patentschriften 4,689,205 sowie 4,579,999 kommt hierbei in der ersten Verfahrensstufe ein Wirbelbettreaktor zum Einsatz. Außerdem wird vom Benzinprodukt ein Teilstrom als ungesättigter Kohlenwasserstoffstrom zur ersten und zur zweiten Verfahrensstufe recycelt. Zur ersten Verfahrensstufe wird zusätzlich Ethylen und Ethan recycelt.

In der dritten US-Patentschrift 4,898,717 wird in beiden Verfahrensstufen ein mehrstufiger Festbettreaktor eingesetzt. Die erste Verfahrensstufe wird hier ohne Rezirkulierung betrieben. Aus dem MTO-Reaktorprodukt der ersten Verfahrensstufe wird als Nebenprodukt ein C₂-Strom separiert, aus dem Ethylen gewonnen wird. Dies ist in diesem Verfahren sinnvoll, da bei den beschriebenen Betriebsbedingungen ca. 26 Gew.-% des MTO-Reaktorprodukts aus Ethylen besteht. Dies reduziert offensichtlich den Gesamtumsatz von Methanol zu Diesel.

Sowohl das MTP-Verfahren als auch das mit Wirbelbettreaktor betriebene MTO-Verfahren sind dadurch gekennzeichnet, dass man die Olefinerzeugung in der ersten Verfahrensstufe in Gegenwart eines Gasstromes durchführt, der angereichert ist mit ungesättigten Kohlenwasserstoffen, die aus dem Produktstrom der ersten oder der zweiten Verfahrensstufe abgetrennt und wieder der ersten Verfahrensstufe zugeführt werden. Damit erreicht man beim MTP-Verfahren, dass die Ausbeute an Propylen optimiert wird. Beim MTO-MOGD-Verfahren werden ungesättigte Kohlenwasserstoffe aus dem Nebenprodukt Benzin rezirkuliert, wodurch die Produktausbeute an Diesel erhöht und die Produktausbeute an Benzin reduziert wird.

Die Rezirkulierung olefinreicher Ströme zum Olefin-Reaktor führt allerdings dazu, dass die Olefine teilweise zu Paraffinen und sonstigen Komponenten umgesetzt werden. Paraffine und sonstige Komponenten stehen im Oligomerisierungs-Reaktor nicht mehr für die Bildung langkettigerer Kohlenwasserstoffe zur Verfügung, was im Endeffekt die Dieselausbeute reduziert.

### DARSTELLUNG DES ERFINDUNGSGEMÄSSEN VERFAHRENS

Ziel des erfindungsgemäßen Verfahrens ist es, ein von Methanol und/oder Dimethylether und/oder einem anderen Oxigenat ausgehendes Verfahren zur Herstellung von synthetischen Kraftstoffen zu entwickeln, bei dem die Dieselausbeute deutlich erhöht wird.

Gelöst wird diese Aufgabe durch ein Verfahren zur Herstellung von synthetischen Kraftstoffen, bei dem
- in einer ersten Verfahrensstufe ein aus Methanol und/oder Dimethylether und oder einem anderen Oxigenat sowie Wasserdampf bestehendes Gasgemisch bei Temperaturen zwischen 300 und 600°C zu Olefinen mit vorzugsweise 2 bis 8 Kohlenstoffatomen umgesetzt wird und
- in einer zweiten Verfahrensstufe das erhaltene Olefingemisch bei erhöhtem Druck zu höheren Olefinen mit überwiegend mehr als 5, vorzugsweise 10 bis 20 Kohlenstoffatomen, oligomerisiert wird.

Erfindungsgemäß wird dabei in der ersten Verfahrensstufe Olefine in Gegenwart eines Gasstromes erzeugt, der im wesentlichen aus gesättigten Kohlenwasserstoffen besteht, welche aus dem Produktstrom der zweiten Verfahrensstufe abgetrennt und wieder der ersten Verfahrensstufe zugeführt werden. Anschließend wird dann in einer zweiten Verfahrensstufe die Olefinerzeugung in Gegenwart eines Wasserdampfstromes durchgeführt, welcher aus dem Produktstrom der ersten Verfahrensstufe abgetrennt und wieder der ersten Verfahrensstufe zugeführt wird.

Diese Lösung beruht auf der Erkenntnis, dass die Produktausbeute an Diesel sowohl durch die Rezirkulierung eines Gasstroms, der überwiegend aus gesättigten Kohlenwasserstoffen besteht, als auch durch die Rezirkulierung von Wasser zur ersten Verfahrensstufe deutlich erhöht wird.

Die Rezirkulierung von Wasser zur ersten Verfahrensstufe reduziert hierbei die Verkokung des Katalysators. Beide Rezirkulationsströme zur ersten Verfahrensstufe, also Wasser und Kohlenwasserstoffe, reduzieren den Partialdruck der reaktiven Komponenten und die adiabate Temperaturerhöhung im Reaktor, was die Selektivität des Katalysators zu den gewünschten Produkten verbessert.

Der zur ersten Verfahrensstufe rezirkulierte Kohlenwasserstoffstrom besteht überwiegend aus gesättigten Kohlenwasserstoffen mit 2 bis 8 Kohlenstoffatomen, vorzugsweise 3 bis 5 Kohlenstoffatomen, und wird in der Separation nach der zweiten Verfahrensstufe aus dem Reaktorprodukt der zweiten Verfahrenstufe abgetrennt.

Der rezirkulierte Wasserstrom besteht aus einem Teilstrom des Reaktionswassers, welches in der Separation nach der ersten Verfahrensstufe vom Reaktorprodukt der ersten Verfahrensstufe abgetrennt wird. Zusätzlich kann eine leichte Fraktion mit weniger als 3 Kohlenstoffatomen aus dem Produkt der ersten Verfahrensstufe abgetrennt werden, und zumindest teilweise zum Einsatzstrom der ersten Verfahrensstufe recycelt werden. Weiter ist es prinzipiell möglich nach der ersten Verfahrensstufe eine Benzinfraktion zu separieren. Dies würde allerdings die Dieselausbeute reduzieren.

Die in dem Reaktionsprodukt der ersten Verfahrensstufe enthaltenen Olefine werden in der zweiten Verfahrensstufe durch Oligomerisierung weitgehend zu höheren Kohlenwasserstoffen umsetzt.

In der anschließenden Separation werden zwei Kohlenwasserstoffströme bereitgestellt, von denen der erste vorzugsweise aus gesättigten Kohlenwasserstoffen besteht und zur ersten Verfahrensstufe rezirkuliert wird. Der zweite Kohlenwasserstoffstrom unterscheidet sich in seiner Zusammensetzung vom ersten, und wird zur zweiten Verfahrensstufe rezirkuliert. Außerdem werden die Produkte Diesel, Benzin, LPG und Heizgas separiert, wobei Diesel aus der schweren Fraktion des Reaktorproduktes durch Hydrierung gewonnen wird.

Sowohl der Olefin-Reaktor als auch der Oligomerisierungs-Reaktor wird als mehrstufiger adiabater Festbettreaktor ausgeführt, wobei verschiedene Ausführungen der Reaktoren möglich sind, wie zum Beispiel Schichtreaktor, Radialreaktor, Rohrreaktor oder andere. Bei diesem Verfahren wird sowohl zur Olefinerzeugung als auch zur Oligomerisierung vorzugsweise ein Zeolith-Katalysator vom Pentasiltyp eingesetzt. Dieser Katalysator weist vorzugsweise ein Si/AI-Atomverhältnis größer 10, auf und hat eine BET-Oberfläche von 300 bis 600 m²/g sowie ein Porenvolumen (bestimmt nach der Quecksilber-Porosimetrie) von 0,3 bis 0,8 cm³/g. Abweichend von dem hier genannten Aluminiumsilikat-Katalysatortyp können auch Katalysatoren basierend auf Silikalit-Material oder Aluminiumphosphat (SAPO Typ) eingesetzt werden. Darüber hinaus kann der Katalysator entweder in Form von Extrudaten oder in Form eines mit Zeolithen beschichteten keramischen Trägers vorliegen.

Das erfindungsgemäße Verfahren wird in der ersten Verfahrensstufe bei einer Temperatur zwischen 300 °C bis 600 °C, vorzugsweise zwischen 400 °C bis 500 °C, insbesondere zwischen 450 °C bis 480°C und bei einem Druck von 0 bis 12 bar , vorzugsweise 1 bis 5 bar , insbesondere bei 1 bis 2 bar durchgeführt.

Das verbleibende Reaktionsprodukt der ersten Verfahrensstufe wird in der zweiten Verfahrensstufe einer Oligomerisierung zugeführt. Durch die Oligomerisierung der Olefine ist das Reaktionsprodukt der zweiten Verfahrensstufe weitgehend von C₃ bis C₅ Olefinen befreit. Bei einer Rezirkulierung eines aus dem Reaktorprodukt des Oligomerisierungs-Reaktors separierten kurzkettigen Kohlenwasserstoffstroms (C₉ und kürzer, vorzugsweise C₅ und kürzer) werden daher nur geringe Mengen Olefine in die erste Verfahrensstufe zurückgeführt. Hierdurch wird ein Verlust an Olefinen weitgehend vermieden, der auch eine Verminderung der Ausbeute an synthetischem Diesel in der zweiten Verfahrenstufe zur Folge hätte.

Im Gegensatz zur Rezirkulierung gesättigter Kohlenwasserstoffe zum Olefin-Reaktor kann die Ausbeute des Oligomerisierungs-Reaktors durch Rezirkulierung ungesättigter Kohlenwasserstoffe optimiert werden. Die Qualität des Kraftstoffes wird dabei maßgeblich durch die Einstellung des Olefin/Paraffin-Verhältnisses in dem der Oligomerisierung zugeführten Gasstrom beeinflusst. Durch Regulierung dieses Mischungsverhältnisses lassen sich die Eigenschaften des so hergestellten synthetischen Kraftstoffes weitgehend beeinflussen.

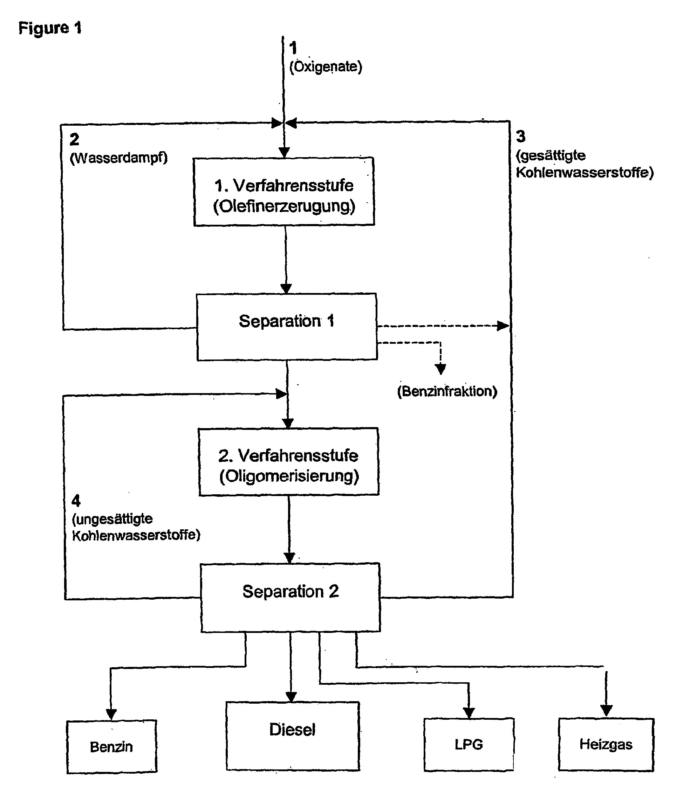

Fig. 1 zeigt in einer schematischen Übersicht die wichtigsten Stufen des erfindungsgemäßen Verfahrens:

Das zur Kraftstoffherstellung eingesetzte Methanol wird verdampft und ggf. daraus teilweise Dimethylether hergestellt. Dieses Gasgemisch 1 wird nach Einleitung von Wasserdampf 2 und einem Gasstrom 3, der vorzugsweise leichte Paraffine enthält, in der 1. Verfahrensstufe (Olefinerzeugung) bei Temperaturen von 300 bis 600 °C, vorzugsweise 450 bis 480 °C, und einem Druck von 0 bis 12 bar, vorzugsweise 1 bis 2 bar, in Gegenwart von Zeolith-Katalysatoren vom Pentasiltyp zu einem Olefingemisch umgesetzt. Die hierbei entstehenden Olefine weisen vorzugsweise 2 bis 9 Kohlenstoffatome auf. Das in der ersten Verfahrensstufe produzierte Wasser wird aus dem Reaktorprodukt abgetrennt und teilweise rezirkuliert. Anschließend kann zur Optimierung der Ausbeute eine leichte Fraktion (C₂) abgetrennt werden und teilweise zur ersten Verfahrensstufe rezirkuliert werden. Besonders vorteilhaft ist es, wenn das molare Verhältnis der gesättigten Kohlenwasserstoffe im Recyclestrom 3 zu den Oxigenaten im Einsatzstrom 1 zwischen 0,5 und 10 liegt.

Das verbleibende Kohlenwasserstoffgemisch wird dann in die 2. Verfahrensstufe (Oligomerisierung) überführt, wo bei Temperaturen zwischen vorzugsweise 200 bis 450 °C und bei einem Druck von 40 bis 100 bar ebenfalls in Gegenwart von Zeolith-Katalysatoren vom Pentasiltyp die Oligomerisierung erfolgt. Die durch Oligomerisierung entstehenden Olefine weisen überwiegend mehr als 5, vorzugsweise zwischen 10 bis 20 Kohlenstoffatome auf. Das erhaltene Gemisch wird in einer anschließenden Separation aufgearbeitet, wobei ein Destillat abgetrennt wird, welches nach Hydrierung das Hauptprodukt Diesel bildet. Weiter werden zwei Kohlenwasserstoffströme unterschiedlicher Zusammensetzung bereitgestellt, von denen der Strom 3 zur ersten Verfahrensstufe und der Strom 4 zur zweiten Verfahrensstufe rezirkuliert wird. Besonders vorteilhaft ist es, wenn das molare Verhältnis von Wasserdampf im Recyclestrom 3 zu den Oxigenaten im Einsatzstrom 1 zwischen 0,1 und 5 liegt.

Schließlich werden die Nebenprodukte Benzin und LPG sowie Heizgas separiert.

Zum experimentellen Nachweis der Erhöhung der Olefinausbeute durch das erfindungsgemäße Verfahren wurde entsprechend dem Stand der Technik dem Einsatzstrom des Olefin-Reaktors, bestehend aus Methanol, Dimethylether und Wasser, ein C₅-Olefinstrom zudosiert. In dem erhaltenen Produkt wurden die entstandenen Mengen von Propylen, Gesamtolefinen, Paraffinen, Naphthenen und Aromaten bestimmt.

Zum Vergleich wurde dann bei dem erfindungsgemäßen Verfahren die Gaszusammensetzung des erhaltenen Produktstromes bestimmt, wobei ein weitgehend inertes Paraffinträgergas in der ersten Verfahrensstufe eingesetzt wurde. Auch bei dieser Betriebsweise des Olefin-Reaktors wurde die Menge des erhaltenen Propylens sowie die Menge der Gesamtolefine, außerdem aber auch die Menge der entstandenen Paraffine, Naphthene und Aromaten bestimmt. Das Ergebnis ist in der folgenden Tabelle 1 dargestellt. Angegeben ist hier die Kohlenstoffausbeute bezogen auf 100 Teile Kohlenstoff im eingesetzten Methanol.

**Tabelle 1**

| | **Propylen** | **Olefine** | **Paraffine** | **Naphthene** | **Aromaten** | **Andere** |
|---|---|---|---|---|---|---|
| **Olefin-Recycling** | 63,1 | 65,1 | 18,2 | 2,5 | 10,3 | 3,9 |
| **Erfindungsgemäßes Verfahren** | 37,9 | 807 | 10,6 | 0,7 | 5,1 | 2,9 |

Zur Berechnung der Umsätze in der Tabelle wurden die Mole des rezirkulierten Olefinstroms bzw. des rezirkulierten Paraffinträgergases sowohl vom Reaktoreinsatz als auch vom Reaktorprodukt abgezogen, da diese bezogen auf das Gesamtverfahren interne Kreislaufströme sind.

Die Tabelle zeigt also den Methanolumsatz mit oder ohne Rezirkulierung von Olefinen. Deutlich erkennbar ist dabei die Optimierung der Propylenausbeute bei Durchführung einer Rezirkulierung von Olefinen. Demgegenüber ist beim erfindungsgemäßen Verfahren die gebildete Propylenmenge vermindert, dafür aber die Gesamtausbeute an Olefinen mit 80,7 besonders hoch.

Im Gegensatz zum Stand der Technik werden bei dem erfindungsgemäßen Verfahren durch die bevorzugte Rezirkulierung von Paraffinen und die Rezirkulierung von Wasser die Reaktionsbedingungen im Festbettreaktor der 1. Verfahrensstufe bezüglich Temperaturprofil und Partialdruck der reaktiven Komponenten so geändert, dass mehr reaktionsfähige Olefine zum Oligomerisierungs-reaktor gelangen, wodurch die Dieselausbeute erheblich vergrößert wird.

Die Prozessstufen Olefinerzeugung und Oligomerisierung wurden in Labor- und Technikumsversuchen über 10000 Stunden bei verschiedenen Betriebsbedingungen untersucht. Die Auswertung dieser Versuche ermöglichte eine Prozesssimulation der drei Verfahrensschritte Olefinerzeugung, Oligomerisierung und Separation.

## Patentansprüche

1. Verfahren zur Herstellung von synthetischen Kraftstoffen, bei dem
- in einer ersten Verfahrensstufe ein aus Methanol und/oder Dimethylether und/oder einem anderen Oxigenat sowie Wasserdampf bestehendes Gasgemisch bei Temperaturen zwischen 300 und 600°C zu Olefinen mit vorzugsweise 2 bis 8 Kohlenstoffatomen umgesetzt und
- in einer zweiten Verfahrensstufe das erhaltene Olefingemisch bei erhöhtem Druck zu höheren Olefinen mit überwiegend mehr als 5, vorzragsweise 10 bis 20 Kohlenstoffatomen, oligomerisiert wird,
**dadurch gekennzeichnet, dass** man
a) die Olefinerzeugung in der ersten Verfahrensstufe in Gegenwart eines Gasstromes durchführt, der im wesentlichen, aus gesättigten Kohlenwasserstoffen besteht, welche aus dem Produktstrom der zweiten Verfahrensstufe abgetrennt und wieder der ersten Verfahrensstufe zugeführt werden und außerdem
b) der ersten Verfahrensstufe einen Wasserdampfström zuführt, welcher anschließend aus dem Produktstrom der ersten Verfahrensstufe wieder abgetrennt und dann der ersten Verfahrensstufe erneut zugeführt wird,

2. Verfahren nach Anspruch 1, **dadurch gekennzeichnet, dass** man sowohl zur Olefinerzeugung als auch zur Oligomerisierung einen Zeolith-Katalysator vom Pentasiltyp oder Katalysatoren basierend auf Silikalit oder Aluminiumphosphat (SAPO Typ) einsetzt.

3. Verfahren nach den Ansprüchen 1 bis 2, **dadurch gekennzeichnet, dass** man als Zeolith-Katalysator einen Protonen enthaltenden Katalysator vom Pentasiltyp mit einem Si/Al-Atomverhältnis größer 10, einer BET-Oberfläche von 300 bis 600 m²/g und einem Porenvolumen ( bestimmt nach der Quecksilber-Porosimetrie ) von 0.3 bis 0,8 cm³/g einsetzt.

4. Verfahren nach den Ansprüchen 1 bis 3, **dadurch gekennzeichnet, dass** die Olefinerzeugung bei 300 bis 600 °C und bei einem Druck von 0 bis 12 bar erfolgt.

5. Verfahren nach den Ansprüchen 1 bis 4, **dadurch gekennzeichnet, dass** die Oligomerisierung bei einer Temperatur von 200 bis 450°C und bei einem Druck von 40 bis 100 bar erfolgt.

6. Verfahren nach den Ansprüchen 1 bis 5, **dadurch gekennzeichnet, dass** die Reaktoren der ersten und zweiten Verfährensstufe aus einem oder mehreren adiabate Festbettreaktoren mit einer oder mehreren Stufen, parallel oder in Reihe, bestehen.

7. Verfahren nach den Ansprüchen 1 bis 6, **dadurch gekennzeichnet, dass** das molare Verhältnis der gesättigten Kohlenwasserstoffe im Recyclestrom 3 zu den Oxigenaten im Einsatzstrom 1 zwischen 0.5 bis 10 liegt.

8. Verfahren nach den Ansprüchen 1 bis 7, **dadurch gekennzeichnet, dass** der zur ersten Verfahrensstufe rezirkulierte Gasstrom überwiegend aus gesättigten C₂ bis C₉ Kohlenwasserstoffen besteht.

9. Verfahren nach den Ansprüchen 1 bis 8, **dadurch gekennzeichnet, dass** das molare Verhältnis von Wasserdampf im Recyclestrom 2 zu den Oxigenaten im Einsatzstrom 1 zwischen 0,1 und 5 liegt.

10. Verfahren nach den Ansprüchen 1 bis 9, **dadurch gekennzeichnet, dass** der zur zweiten Verfahrensstufe rezirkulierte Gasstrom sich in seiner Zusammensetzung von dem zur ersten Verfahrensstufe rezirkulierten Gasstrom unterscheidet.

## Claims

1. A method for the manufacture of synthetic fuels, in which
- In a first method stage a gas mixture comprised of methanol and/or dimethylether and/or another oxygenate such as steam reacts at temperatures between 300 and 600 °C to olefins preferably having 2 to 8 carbon atoms, and
- In a second method stage the olefin mixture obtained is oligomerized to higher olefins with predominantly more than 5, preferably 10 to 20 carbon atoms,
**characterized in that**
a) the olefin production in the first method stage is carried out in the presence of a gas stream, which essentially comprises saturated hydrocarbons, which are separated from the product stream of the second method stage and are fed again to the first method stage, and
b) the olefin production in the second method stage is carried out in the presence of a steam stream, which is separated from the product stream of the first method stage and fed again to the first method stage.

2. The method according to claim 1
**characterized in that**
as for the olefin production as well as for the oligomerization a zeolite catalyst of the pentasil type or catalysts based on silikalit or aluminum phosphate (SAPO type) are employed.

3. The method according to claims 1 to 2,
**characterized in that**
as zeolite catalyst a proton-containing catalyst of the pentasil type having an Si/Al ratio larger than 10, a BET surface area of 300 tro 600 m²/g and a pore volume (determined by mercury porosimetry) of 0.3 to 0.8 cm³/g is employed.

4. The method according to claims 1 through 3,
**characterized in that**
the olefin production is carried out at 300 to 600 °C and at a pressure of 0 to 12 bar.

5. The method according to claims 1 through 4,
**characterized in that**
the oligomerization is carried out at a temperature of 200 to 450 °C and at a pressure of 40 to 100 bar.

6. The method according to claims 1 through 5,
**characterized in that**
the reactors of the first and second method stage comprise one or a plurality of adiabatic solid bed reactors, having one or a plurality of stages, parallel or in series.

7. The method according to claims 1 through 6,
**characterized in that**
the molar ratio of saturated hydrocarbons, in the recycle stream 3 to the oxygenates in input stream 1 lies between 0.5 and 10.

8. The method according to claims 1 through 7,
**characterized in that**
the gas stream recirculated to the first method stage comprises predominantly saturated C₂ to C₉ hydrocarbons.

9. The method according to claims 1 through 8,
**characterized in that**
the molar ratio of steam in recycle stream 2 to the oxygenates in the input stream 1 lies between 0.1 and 5.

10. The method according to claims 1 through 9,
**characterized in that**
the gas stream recirculated to the second method stage differs in its composition from that to the gas stream recirculated to the first method stage.

## Revendications

1. Procédé de fabrication de carburants synthétiques, dans lequel
- dans une première étape du procédé, un mélangé gazeux constitué de méthanol et/ou d'éther diméthylique et/ou d'un autre produit oxygéné ainsi que de vapeur d'eau subit une réaction à des températures comprises entre 300 et 600 °C, afin d'obtenir des oléfines comprenant, de préférence, 2 à 8 atomes de carbone et,
- dans une deuxième étape du procédé, le mélange d'oléfines obtenu est oligomérisé sous une pression élevée afin d'obtenir des oléfines plus lourdes comprenant majoritairement plus de 5, de préférence 10 à 20, atomes de carbone,
**caractérisé en ce qu'**on
a) réalise la préparation des oléfines dans la première étape du procédé en présence d'un flux gazeux constitué essentiellement d'hydrocarbures saturés qui ont été séparés du flux des produits issus de la deuxième étape du procédé pour être réintroduits dans la première étape du procédé et, en outre,
b) introduit un flux de vapeur d'eau dans la première étape du procédé lequel est ensuite séparé du flux des produits issus de la première étape du procédé puis réintroduit dans la première étape du procédé.

2. Procédé selon la revendication 1, **caractérisé en ce que**, tant pour la préparation des oléfines que pour l'oligomérisation, on met en oeuvre un catalyseur zéolithique de type pentasil ou des catalyseurs à base de silicalite ou de phosphate d'aluminium (de type SAPO).

3. Procédé selon les revendications 1 à 2, **caractérisé en ce que**, en tant que catalyseur zéolithique, on met en oeuvre un catalyseur de type pentasil contenant des protons et présentant un rapport atomique Si/Al supérieur à 10, une surface BET comprise entre 300 et 600 m²/g et un volume poreux (déterminé par porosimétrie au mercure) compris entre 0,3 et 0,8 cm³/g.

4. Procédé selon les revendications 1 à 3, **caractérisé en ce que** la préparation des oléfines est réalisée entre 300 et 600 °C et sous une pression comprise entre 0 et 12 bar.

5. Procédé selon les revendications 1 à 4, **caractérisé en ce que** l'oligomérisation est réalisée à une température comprise entre 200 et 450 °C et sous une pression comprise entre 40 et 100 bar.

6. Procédé selon les revendications 1 à 5, **caractérisé en ce que** les réacteurs de la première et de la deuxième étape du procédé sont constitués par un ou plusieurs réacteurs adiabatiques à lit fixe comprenant un ou plusieurs étages disposés en parallèle ou en série.

7. Procédé selon les revendications 1 à 6, **caractérisé en ce que** le rapport molaire entre les hydrocarbures saturés dans le flux de recyclage 3 et les produits oxygénés dans le flux d'alimentation 1 est compris entre 0,5 et 10.

8. Procédé selon les revendications 1 à 7, **caractérisé en ce que** le flux gazeux recyclé vers la première étape du procédé est majoritairement constitué d'hydrocarbures saturé en C₂ à C₉.

9. Procédé selon les revendications 1 à 8, **caractérisé en ce que** le rapport molaire entre la vapeur d'eau dans le flux de recyclage 2 et les produits oxygénés dans le flux d'alimentation 1 est compris entre 0,1 et 5.

10. Procédé selon les revendications 1 à 9, **caractérisé en ce que** la composition du flux gazeux recyclé vers la deuxième étape du procédé est différente de celle du flux gazeux recyclé vers la première étape du procédé.
